(19) Europäisches Patentamt
European Patent Office
Office européen des brevets

(11) Veröffentlichungsnummer: **0 235 526**
**A2**

(12) # EUROPÄISCHE PATENTANMELDUNG

(21) Anmeldenummer: **87100714.2**

(22) Anmeldetag: **20.01.87**

(51) Int. Cl.⁴: **C08F 8/42**, C12N 11/08, C08G 85/00, C08J 7/12

(30) Priorität: **29.01.86 DD 286581**
**29.01.86 DD 286582**

(43) Veröffentlichungstag der Anmeldung:
**09.09.87 Patentblatt 87/37**

(84) Benannte Vertragsstaaten:
**AT BE DE ES FR GB NL SE**

(71) Anmelder: **VEB Leipziger Arzneimittelwerk**
**Elisabeth-Schumacher-Strasse 54-56**
**DDR-7050 Leipzig(DD)**

(72) Erfinder: **Schössler, Werner, Dr.**
**Rathenaustrass 12**
**DDR-1297 Neu-Buch(DD)**
Erfinder: **Boeden, Hans-Friedrich, Dr.**
**Brehmestrasse 50**
**DDR-1100 Berlin(DD)**
Erfinder: **Holtzhauer, Martin, Dr.**
**Wandlitzstrasse 13**
**DDR-1157 Berlin(DD)**
Erfinder: **Loth, Fritz, Dr.**
**Ernst-Thälmannstrasse 211**
**DDR-1530 Teltow(DD)**
Erfinder: **Hiepe, Falk, Dr.**
**Zum Langen See 22**
**DDR-1178 Berlin(DD)**
Erfinder: **Mielke, Frank, Dr.**
**Oberseestrasse 46**
**DDR-1092 Berlin(DD)**
Erfinder: **Bertram, Dieter, Dr.**
**Strasse des 18 Oktober 8/75**
**DDR-7010 Leipzig(DD)**
Erfinder: **Müller, Reinhard, Dr.**
**Krönestrasse 48**
**DDR-7050 Leipzig(DD)**
Erfinder: **Konjuchowa, Dagmar**
**Erich-Köhnen-Strasse 65**
**DDR-7033 Leipzig(DD)**

(74) Vertreter: **Puchberger, Rolf, Dipl. Ing. et al**
**Patentanwälte, Dipl. Ing. Georg Puchberger**
**Dipl. Ing. Rolf Puchberger Dipl. Ing. Peter**
**Puchberger Singerstrasse 13 Postfach 55**
**A-1010 Wien(AT)**

(54) **Aktivierte Polymerfestkörper und Verfahren zu ihrer Herstellung.**

EP 0 235 526 A2

Xerox Copy Centre

(57) Die Erfindung betrifft chemisch modifizierte und aktivierte Hydroxyl-Gruppen enthaltende natürliche und synthetische polymere Festkörperoberflächen sowie ein Verfahren zur Aktivierung von Hydroxyl-Gruppen enthaltenden synthetischen und natürlichen polymeren Festkörperoberflächen mit Organosilanen und gegebenenfalls homo-oder heterobifunktionellen Reagenzien zur einfachen, schonenden und stabilen Bindung von Proteinen, Nukleinsäuren, niedermolekularen Liganden, Zellen, Mikroorganismen und anderen biologischen Materialien.

Die Erfindung findet Anwendung in den Biowissenschaften, der Biotechnologie und in der Medizin.

Die Erfindung betrifft chemisch modifizierte und aktivierte Hydroxyl-Gruppen enthaltende natürliche und synthetische polymere Festkörperoberflächen, die für die Bindung von Proteinen, Nukleinsäuren, niedermolekularen Liganden, Zellen, Mikroorganismen und anderen biologischen Materialien in der Biotechnologie, den Biowissenschaften sowie der Medizin für analytische und präparative Zwecke eingesetzt werden und ein Verfahren zu ihrer Herstellung.

Trägergebundene biologisch aktive Substanzen finden seit Jahren breite Anwendung zur Separation und Isolierung spezifischer Wechselwirkungsparnter (Übersicht: H.D. Orth, W. Brümer; Angew.Chem.84 - (1972) 319; W.B. Jakob, M. Wilchek (Hrsg.): Methods in Enzymol. 34(1974), zur Bindung von Enzymen und Einsatz derselben als Enzymreaktoren (K. Mosbach (Hrsg.): Methods in Enzymol.44 (1976), sowie zum qualitativen und quantitativen Nachweis von biologisch bedeutsamen Verbindungen in den Biowissenschaften, der Biotechnologie, aber auch zunehmend in der Medizin.

Generell sind an eine ideale Matrix folgende Anforderungen zu stellen:

-Unlöslichkeit
-Makroporosität
-mechanische und chemische Stabilität
-partikuläre Form
-Hydrophilität
-geringe unspezifische Bindung
-Resistenz gegenüber mikrobiellen und enzymatischen Einflüssen
-Vorhandensein funktioneller Gruppen für eine chemische Modifizierung und Aktivierung.

Eine derartige ideale Matrix, die allen Anforderungen gerecht wird und somit universell einsetzbar ist, existiert nicht. Deshalb wurden in den letzten Jahren eine Vielzahl von Trägermaterialien beschrieben und kommerziell angeboten, wobei deren Palette von natürlichen Polymeren (Polysacchariden), wie Dextranen und Agarose, bis zu synthetischen Polymeren, wie Polyvinylalkoholen, Acrylsäurederivaten, Vinyl-Polymeren, sowie Copolymeren aus natürlichen und synthetischen Polymeren, wie Agarose und Polyacrylamid, reicht. Alle diese Träger besitzen mehr oder weniger Hydroxyl-Gruppen auf ihrer Oberfläche, die wesentlich für die hydrophilen Eigenschaften und somit geringen unspezifischen Bindungen verantwortlich sind. Voraussetzung für die Bindung von biologisch aktiven Substanzen wie Proteinen, Lektinen, Enzymen, Nukleinsäuren, niedermolekularen Liganden, Zellen und anderen biologischen Materialien ist in vielen Fällen das Einführen chemisch reaktiver Gruppen, die eine chemische Bindung ermöglichen. Die als Aktivierung bezeichnete chemische Modifizierung hängt prinzipiell von der Art der funktionellen Gruppen der Matrix und des Liganden ab, wird aber auch von der chemischen Stabilität der Matrix sowie der Stabilität der biologischen Eigenschaften des Liganden determiniert. Die Wahl des Aktivierungsverfahrens wird des weiteren durch den technologischen Aufwand bei der Aktivierung und die hiemit verbundenen Kosten des Aktivierungsverfahrens, der Reaktivität der eingeführten chemischen Gruppen, der Möglichkeit der Lagerung des aktivierten Trägers, der Toxizität und Biokompatibilität der Modifizierungsreagenzien sowie der Stabilität der chemischen Bindung zwischen Festkörperoberfläche und Ligand bestimmt.

Deshalb nimmt es bei der Vielzahl der zu beachtenden und limitierenden Parameter und Einflußfaktoren nicht wunder, daß in Verbindung mit den zahlreichen Trägermaterialien eine kaum noch zu iberschauende Zahl an Aktivierungsverfahren beschrieben wurde. Stellvertretend hierfür sollen die Aktivierungsverfahren mit Glutaraldehyd oder anderen homo-oder heterobifunktionellen Reagenzien, die CNBr-Aktivierung, die Aktivierung mit Hydrazin, Bisepoxiranen, Divinylsulfon, Epichlorhydrin, Benzochinon, Carbonylimidazolen, Triazin-Derivaten, Tosylchloriden sowie die Perjodat-Oxidation und Diazotierung genannt werden (Übersicht: J.M. Egly, E. Boschetti: Practical guide for use in affinity chromotography and related techniques, IBF-LKB, 1983).

Mit Abstand am verbreitesten ist die durch Axen et al. eingeführte Aktivierung mittels Bromcyan (R. Axen, J.Porath, S. Ernback: Nature 214 (1967) 1302). Obwohl dieses Verfahren unstrittig zu einer breiten Anwendung trägerfixierter Substanzen in den Biowissenschaften und der Biotechnologie führte, ist es doch mit den Nachteilen einer relativ instabilen chemischen Bindung zwischen Festköperoberfläche und Ligand (insbesondere bei ph-Werten < 5 und >10, die zu einer nicht unbeträchtlichen Freisetzung des gebundenen Liganden (leakage) führen kann und somit die Anwendung vor allem für therapeutische in vivo-Verfahren in der Medizin stark eingeschränkt, der hohen Toxizität von Bromcyan, die die Technologie der Aktivierung aufwendig gestaltet, und der Gefahr der Ausbildung kationischer Gruppen am Träger behaftet.

Nachteilig bei der Hydrazin-Aktivierung sind die hierbei auftretenden technischen Probleme sowie die niedrigen Kopplungsausbeuten mit hochmolekularen Liganden.

Andere Aktivierungsverfahren hingegen sind mit den Nachteilen einer hohen Toxizität der Reagenzien - (Divinylsulfon, Triazin-Derviate, Epichlorhydrin) und/oder hohen Kosten des Verfahrens (Divinylsulfon, Tosylchlorid, Tresylchlorid), ungünstigen Milieubedingungen während der Kopplung und somit Gefahr einer Inaktivierung biologischer Materialien (Bisepoxirane, Triazin-Derivate, Epichlorhydrin), einer instabilen Bindung bei höheren pH -Werten (Divinylsulfon, Carbonyldiimidazol, Diazoniumverbindungen), langen Aktivierungs-und Bindungszeiten für Liganden (Epichlorhydrin, Bisepoxirane), technologischen Problemen, die wiederum Einfluß auf die Kosten des Verfahrens haben (Triazin-Derivate, Tosylchlorid, Carbonyldiimidazol, Diazotierung), niedrigen Kopplungsausbeuten (Perjodat-Oxidation), sowie relativ starken -auf charge transfer -Wechselwirkungen zurückzuführende -unspezifischen Bindungen (Benzochinone, Triazin-Derivate, Diazonium-Verbindungen) behaftet.

Es ist seit einiger Zeit bekannt (R.A. Mesing, H.H. Weetall: US-Patent 35 19 538; H.H. Weetall, N.Y. Elmira : US-Patent 36 52 761; H.H. Weetal Methods in Enzymol. 44 (1976) 134), daß die OH-Gruppen auf $SiO_2$ -Oberflächen, vorzugsweise Glas, als Angriffspunkt für chemische Agenzien, insbesondere Organosilanen mit unterschiedlichen funktionellen Gruppen, zur Verfügung stehen. Allerdings wurden diese Organosilane bisher ausschließlich for anorganische Träger und Festkörperoberflächen eingesetzt.

Es ist das Ziel der Erfindung, ein einfaches kostengünstiges, nicht toxisches Aktivierungsverfahren für Hydroxyl-Gruppen enthaltende natürliche und synthetische polymere Festkörperoberflächen zu entwickeln, das zu stabilen aktivierten Festkörperoberflächen führt, an die Proteine, Nukleinsäuren, niedermolekulare Liganden, Zellen, Mikroorganismen und andere biologische Materialien mit hoher Stabilität und Ausbeute sowie Biokompatibilität gebunden werden können.

Die Erfindung hat die Aufgabe, Hydroxyl-Gruppen enthaltende natürliche und synthetische polymere Festkörperoberflächen mit Verbindungen zu aktivieren, die gewöhnlicherweise zur Bindung von organischen Substanzen an anorganische Stoffe eingesetzt werden, wobei hochaktivierte sowie stabile und biokompatible Polymerfestkörper entstehen sollen. Erfindungsgemäß wird die Aufgabe dadurch gelöst, daß die auf natürlichen oder synthetischen Festkörperoberflächen befindlichen hydroxyl-Gruppen mit einem Organosilan der allgemeinen Formel

$$(X\,R'_{n'})_n\ Si\ R_{4-n} \quad (I)$$

umgesetzt werden, wobei X eine Amino-, Carbonyl-, Carboxyl-, Epoxy-, Isocyano-, Diazo-, Isothiocyano- ,Nitroso-, Sulfhydryl-oder Halocarbonyl-Gruppe und R′ eine Alkyl-, Alkylphenyl-oder Phenyl-Gruppe ist, während R für eine Alkoxy-, Phenoxy-oder Halogen-Gruppe steht und n′ den Wert zwischen 0 und 20 und n den Wert zwischen 1 bis 3 haben kann. Gebräuchliche Organosilane können durch folgende Formel dargestellt werden:

$$Y_n\ Si\ R_{4-n} \quad (II)$$

wo Y eine Amino-, Carbonyl-, Carboxy-, Epoxy-oder Sulfhydryl-Gruppe darstellt und R für eine Alkoxy-, Phenoxy-und Halogen-Gruppe steht und n den Wert zwischen 1 -3 annehmen kann. In der Praxis weit verbreitete und oft genutzte Organosilane besitzen die allgemeine Zusammensetzung:

$$XCH_2CH_2CH_2\text{-}Si\ (OR)_3 \quad (III)$$

wo X die reaktive organische Gruppe entsprechend Formel I ist und R für eine Methyl-oder Ethyl-Gruppe steht. Der Umsatz wird auch mit mindestens zwei Organosilanen der Formel I im Gemisch oder nacheinander durchgeführt. Bevorzugtes Milieu für die Organosilane ist die flüssige oder die gasförmige Phase, wobei die Aktivierung in wässrigen Systemen, Wasser-Lösungsmittelgemischen oder organischen Lösungsmitteln erfolgen kann. Anschließend erfolgt gegebenenfalls eine weitere Behandlung der modifizierten Festkörperoberflächen mit homo-oder heterobifunktionellen Reagenzien.

Die nach dem erfindungsgemäßen Verfahren hergestellten aktivierten Polymerfestkörper, bestehend aus organischen, OH-gruppenhaltigen Makromolekülen, besitzen auf ihrer Oberfläche Gruppierungen der allgemeinen Formel

$$(\text{-O-})_{4-n}\ Si\ (R'_{n'}X)_n \quad (IV)$$

und OH-Gruppen, wobei R' eine Alkyl-, Alkylphenyl-oder Phenyl-Gruppe und X eine Amino-, Carbonyl-, Carboxyl-, Epoxy-, Isocyano-, Diazo-, Isothiocyano-Nitroso-, Sulfhydryl-oder Halocarbonyl-Gruppe ist und n' den Wert von 0 -20 und n 1-3 besitzen. Die Gruppierung IV steht zur Anzahl der OH-Gruppen im Verhältnis 1 : 9 bis 9 : 1.

Natürliche und synthetische Polymere besitzen auf ihrer Oberfläche, wie bereits ausgeführt, OH-Gruppen, die die hydrophilen Eigenschaften der Festkörperoberflächen erhöhen und somit zu einer Reduzierung der unspezifischen bzw. unerwünschten Wechselwirkungen beitragen. Obwohl nur ein Bruchteil der in den natürlichen und synthetischen Polymeren vorhandenen OH-Gruppen an der Oberfläche dieser Träger sterisch zugänglich sein sollte, wurde überraschenderweise gefunden, daß Organosilane mit hoher Reaktivität mit derartigen Festkörperoberflächen in hoher Ausbeute reagieren. Während die silikofunktionelle Gruppe mit den zugänglichen Hydroxyl-Gruppen des Festkörpers reagiert, steht die organofunktionelle Gruppe den üblichen chemischen Reaktionen unter Verwendung von Amino-, Carbonyl-, Carboxyl-, Isocyano-, Diazo-, Isothicyano-, Sulfhydryl-, Nitroso-oder Halocarbonyl-Gruppen zur Verfügung, so daß diese Verbindungen als Brücke zwischen der Festkörperoberfläche und der organischen/biologischen Verbindung fungieren. Die so aktivierten natürlichen oder synthetischen Festkörperoberflächen können nunmehr im folgenden direkt über die entsprechenden funktionellen Gruppen oder aber unter Vermittlung von homo-oder heterobifunktionellen Reagenzien auf an sich bekannte Art und Weise mit den zu bindenden Proteinen, Lektinen, Enzymen, Nukleinsäuren, niedermolekularen Liganden, Zellen, Mikroorganismen und anderen biologischen Materialien umgesetzt werden. Das erfolgt beispielsweise erfindungsgemäß so, daß die Hydroxyl-Gruppen enhaltenden Festkörperoberflächen mit $\gamma$-Aminopropyltriethoxysilan umgesetzt werden (X = $NH_2$-; R' = $(-CH_2)_3$; n' = 1; R = $-OC_2H_5$; n = 1 in der Formel I ). Die nunmehr an der Festkörperoberfläche befindliche AminoGruppe kann im folgenden mit Glutardialdehyd oder N-Succinimidyl-3-(2-pyridyldithio)propionat (SDPD) umgesetzt werden. Die Bindung der Proteine oder anderen Liganden erfolgt in diesen Fällen an eine Aldehydgruppe über Amino-Gruppen -vorzugsweise die in E-Stellung befindliche Amino-Gruppe des Lysins -oder über Disulfid-Bindungen.

Ganz analog können die Hydroxy-Gruppen enthaltenden Festkörperoberflächen mit Glycidoxypropyltriethoxysilan umgesetzt werden. Bei dieser Variante reagieren die zu bindenden biologischen Materialien direkt mit der Epoxy-Gruppe der Festkörperoberfläche. Wichtig hierbei ist, daß die Umsetzung mit den Organosilanen, die nicht toxisch sind und großtechnisch in beträchtlichem Umfang produziert werden, durch einfaches in Kontakt bringen oder Tauchen erfolgt, die Aktivierung im gequollenen oder nicht gequollenen Zustand des Festkörpers erfolgen kann oder aber in der Gasphase erfolgt. Von großer Bedeutung hierbei ist, daß die Umsetzung mit Organosilanen in flüssiger Phase mit organischen Lösungsmitteln, wie Aceton, Toluol, Dioxan, Methanol, Ethanol u.a. Lösungsmittelgemischen, wie Methanol/Ethanol, sowie in wässrigem Milieu oder Wasser-Lösungsmittel-Gemischen, wie Ethanol/Wasser und Methanol/Wasser, erfolgen kann, so daß im Gegensatz zu vielen anderen Aktivierungsverfahren der technologische Aufwand gering ist. Besonders vorteilhaft sollte sich die Aktivierung in gasförmiger Phase durch Anwendung von Aerosolen oder durch Unterdruck gestalten lassen. Vorteilhaft ist weiterhin, daß durch Wahl unterschiedlicher Organosilane und gegebenenfalls bifunktioneller Kopplungsreagenzien praktisch alle in Frage kommenden Reaktionsmöglichkeiten realisiert werden können, da unter Nutzung des zugrundeliegenden und erfindungsgemäßen Prinzips Festkörperoberflächen mit den unterschiedlichsten funtkionellen Gruppen (X in Formel I) erhalten werden können. Hinzu kommt, daß -bedingt durch den Spacereffekt der Organosilane (R' = 0 -20) und/oder der bifunktionellen Kopplungsreagenzien (so zB. Glutaraldehyd) -die gebundenen biologischen Materialien fast ausnahmslos ihre biologische Aktivität behalten. Besonders vorteilhaft gestaltet sich der Einsatz von Gemischen an Organosilanen unterschiedlicher funktioneller Gruppen, da diese über unterschiedliche Mechanismen biologische Materialien in hoher Ausbeute binden können. So erweisen sich Gemische aus Aminopropyltriethoxysilan und Glycidoxypropyltriethoxysilan,sowie Aminopropyltriethoxysilan und Mercaptopropyltrimethoxysilan als besonders geeignet, da diese biologischen Materialien durch Reaktion über unterschiedliche funktionelle Gruppen mit sehr hoher Ausbeute binden können. In diesen Fällen erfolgt die Bindung der Liganden iber Aldehyd-und EpoxyGruppen bzw. über Aldehyd-und Mercapto-Gruppierungen.

Durch Einsatz von Mercaptopropyltrimethoxysilan eröffnet sich die Möglichkeit der reversiblen Bindung biologischer Materialien, da die resultierende Disulfid-Bindung durch Anwendung geeigneter Reduktionsmittel reversibel gespalten werden kann. Die reversible Liganden-Kopplung erweist sich insbesondere in solchen Fällen als vorteilhaft, in denen die Elution des spezifisch gebundenenWechselwirkungspartners die Gefahr einer Denaturierung in sich birgt, so daß die Elution des Protein-Ligand-Komplexes vorzuziehen ist.

Die sich durch Reaktion von Organosilanen mit den Hydroxyl-Gruppen der natürlichen und synthetischen Polymere ausbildenden chemischen Bindungen sind sehr stabil, so daß so aktivierte Festkörper im trockenen oder feuchten Zustand über lange Zeit gelagert werden können und daß gebundene biologisch aktive Liganden über lange Zeiträume auch unter extremen Bedingungen stabil gebunden bleiben. Die unspezifischen Bindungen an so aktivierte natürliche oder synthetische polymere Festkörperoberflächen sind äußerst gering. Organosilane zeichnen sich durch eine gute Biokompatibilität aus.

Als natürliche oder synthetische polymere Festkörperoberflächen kommen alle Polymere in Betracht, die über eine ausreichende Zahl sterisch zugänglicher Hydroxyl-Gruppen auf ihrer Oberfläche verfügen, wobei die Zusammensetzung der Festkörper für das erfindungsgemäße Aktivierungsverfahren nicht entscheidend ist. Weit verbreitet sind natürliche Polymere auf der Basis von vernetzten Dextranen - (Sephadex®) oder Polysaccharide auf der Basis von Agarose (Sepharose®) oder Zellulose. Gebräuchliche synthetische Polymere sind z.B. vernetzte Polyvinylalkohole, Copolymere aus Di-, Tri-und Glycidylmethacrylaten mit Alkoholen (Fractogel®) sowie Copolymere, die sich von N-acryloyl-2-amino-2-hydroxymethyl-1,3-propandiol (Trisacryl®) ableiten. Häufig werden jedoch auch Copolymere aus natürlichen und synthetischen Polymeren, wie Agarose und Polyacrylamid (Ultrogel AcA®) eingesetzt, die auch erfindungsgemäß aktiviert werden können.

Festkörper aus Hydroxyethylmethacrylat-Ethylenglykoldimethacrylat-Copolymere sowie zellulosehaltige Festkörper, die über eine ausreichende Zahl sterisch zugänglicher Hydroxyl-Gruppen verfügen, lassen sich ebenfalls mit dem erfindungsgemäßen Verfahren aktivieren.

Die auf diese Art und Weise aktivierten Festkörper liegen zweckmäßigerweise als Formkörper vor, wobei die Formkörper eine sphärische, faserige oder eckige Form besitzen können, die durch Packung, Verweben oder durch Bindemittel zu Kolonnenpackungen, Flächenträgern oder anderen höheren Strukturen verbunden sein können. Eine dominierende Bedeutung hierbei besitzen sphärische zellulosehaltige Festkörper (z.B. Perlzellulose), die vor allem für chromatographische Methoden und Verfahren eingesetzt werden, und flächenförmige Träger ("aktiviertes Papier"), die in der Analytik und Diagnostik eine weite Verbreitung gefunden haben. Eingeschlossen hiervon sind auch dünne Schichten, die als Filme und Lacke auf anderen Festkörpern, die in die erfindungsgemäße Reaktion nicht einbezogen sind, befindlich sind, wobei die für die erfindungsgemäße Aktivierungsreaktion inerten Trägermaterialien homogen oder nicht homogen sowie geformt oder nicht geformt sein können.

Die aktivierten Festkörperoberflächen werden zur chemischen Bindung von Proteinen, wie Antigenen, Antikörpern, Enzymen und Lektinen, Nukleinsäuren, niedermolekularen Liganden, Zellen, Mikroorganismen und anderen biologischen Materialien eingesetzt. Derartige an Festkörperoberflächen gebundene Liganden können zur Separation und Isolierung von biospezifischen Wechselwirkungenspartnern mittels affinitätschromatographischer Verfahren, als Enzymreaktoren, zum qualitativen und quantitativen Nachweis von biologisch bedeutsamen Verbindungen in den Biowissenschaften, der Biotechnologie, aber auch der Medizin Anwendung finden.

Im folgenden wird die Erfindung an einigen Beispielen erläutert:

<u>Beispiel 1</u>

Jeweils 1 g vernetztes Dextran (Sephadex® G 200, Pharmacia, Schweden) Agarose (Sepharose 4 B ®, Pharmacia Schweden) Trisacryl ® (LKB, Schweden) sowie Fractogel ® (Merck, BRD) werden mit 5% Aminopropyltriethoxysilan (NB 1114 VEB Chemiewerk Nünchritz, DDR) in Ethanol/Wasser (1 : 1), pH 2,5; 6 h bei 6o° C inkubiert und anschließend zweimal mit je 5 ml Ethanol/Wasser sowie fünfmal mit je 5 ml 0,1 m Phosphat-Puffer, pH 6,8 gewaschen. Nachfolgend wird der Aktivierungsgrad durch Bestimmung der Aminogruppen an der Festkörperoberfläche nach G. Antoni (Anal.Biochem. 129 (1983) 60) bestimmt. Der Aktivierungsgrad kann durch Wahl der Konzentration der Organosilane und/oder der Inkubationszeiten über einen weiten Bereich variiert werden. In der Regel erhält man charakteristische Aktivierungsgrade von 10 - 30 µMol Aminogruppen je g Träger.

<u>Beispiel 2:</u>

5 ml Trisacryl® GF 200 werden mit 10% Aminopropyltriethoxysilan (NB 1114 VEB Chemiewerk Ninchritz, DDR) in Ethano/Wasser (1 : 1) ph 2,5; 6 Stunden bei 60° C inkubiert und anschließend zweimal mit je 30 ml Ethanol/Wasser (1 : 1) bzw. fünfmal mit je 20 ml 0,1 m Phosphat-Puffer, pH 6,8 gewaschen. Das so aktivierte Gel wird nachfolgend mit 5 %igem Glutaraldehyd 2 Stunden bei 37° C in 0,1 m Phosphat-Puffer, pH 6,8 umgesetzt und nachfolgend wiederum fünfmal mit je 20 ml Phosphat-Puffer gewaschen. Die Bindung des Proteins erfolgt durch einfache Zugabe der Proteinlösung zu dem auf diese Art und Weise aktivierten Gel. Hierzu wird der Träger mit 10 ml humanem Immunglobulin G in einer Konzentration von 35,5 mg/ml in einem 0,1 m Phosphat-Puffer, pH 6,8 2 Stunden bei Raumtemperatur versetzt. Nach Absaugen des nicht gebundenen Überstandes erfolgt die Bestimmung der gebundenen Proteinmenge am Träger durch Differenzmessung der Eiweißkonzentration der eingesetzten Proteinlösung und des Überstandes. Auf diese Art und Weise konnten 49,7 mg IgG/mlTrisacryl gebunden werden.

Beispiel 3:

1 ml Trisacryl® GF 2000 wird, wie in Beispiel 1 und 2 beschrieben, aktiviert und mit Glutaraldehyd in einer Konzentration von 3 % umgesetzt. Anschließend wird der so aktivierte Träger mit $^{125}$J-IgG (vom Kaninchen, gerichtet gegen alkalische Phosphatase) in einer Konzentration von 41,3 mg/ml versetzt und 2 Stunden bei Raumtemperatur inkubiert. Nach Absaugen der überschüssigen Proteinmenge und nachfolgendem intensiven Waschen mit jeweils 5 ml Phosphat-Puffer wird die gebundene Proteinmenge durch Messung der Radioaktivität unter Mitführung der eingesetzten Proteinlösung als Standard bestimmt. In diesem Versuch konnten 32,4 mg $^{125}$J-IgG/ml Trisacryl gebunden werden.

Beispiel 4:

1 ml Trisacryl® 2000 wird mit einem Gemisch aus Aminopropyltriethoxysilan sowie Glycidoxypropyltriethoxysilan (Haftvermittler 6130, VEB Chemiewerk Nünchritz, DDR)in Kontakt gebracht und 2 Stunden bei Raumtemperatur geschüttelt. Nach Absaugen des überschüssigen Reagenz sowie Trocknen und nachfolgendem intensiven Waschen mit 0,1 m Phosphat-Puffer, pH 6,8 wird der Träger mit Glutaraldehyd umgesetzt und anschließend $^{125}$J-IgG, wie in Beispiel 3 beschrieben, gebunden. Es konnten 32,7 mg $^{125}$J-IgG/ml Trisacryl gebunden werden.

Beispiel 5:

1 ml Sepharose 4 B® wird, wie in Beispiel 1 und 2 beschrieben, mit Silanhaftmittel und Glutaraldehyd aktiviert und anschließend mit $^{125}$J-IgG, wie in Beispiel 3 angeführt, in einer Konzentration von 41,3 mg/ml in einem 0,1 m Phosphat-Puffer versetzt und 2 Stunden bei Raumtemperatur inkubiert. Die Menge des gebundenen Proteins, die wie in Beispiel 3 beschrieben, bestimmt wurde, konnte mit 5,6 mg/ml Gel ermittelt werden.

Beispiel 6:

1 ml Sepharose 4 B® wie in Beispiel 4 beschrieben, mit zwei unterschiedlichen Organosilanen und Glutaraldehyd aktiviert und anschließend mit $^{125}$ J-IgG umgesetzt. In diesem Versuch konnten 15,2 IgG/ml Gel gebunden werden.

Beispiel 7:

Die entsprechend den Beispielen 3 und 4 mit $^{125}$J-IgG umgesetzten Gele (Trisacryl) werden je dreimal mit 20 ml PBS-Puffer, pH 7,4,der 20% Dioxan enthält, gewaschen und nachfolgend wird die gebundene Radioaktivität bestimmt. Die am Trisacryl verbliebene Proteinmenge ist mit 14,3 mg/ml unverändert.

Beispiel 8:

Die entsprechend den Beispielen 5 und 6 mit $^{125}$J-IgG umgesetzten Sepharosen werden, wie in Beispiel 7 beschrieben, mit einem PBS-Puffer, der 20 % Dioxan enthält,intensiv gewaschen. Die durch Bestimmung der Radioaktivität nachgewiesenen Proteinmengen wurden mit 5,6 mg/ml Gel (Beispiel 5) bzw. 14,2 mg/ml Gel (Beispiel 6) ermittelt.

Beispiel 9:

Das in den Beispielen 3 -8 eingesetzte Immunglobulin G wurde durch Immunisierung eines Kaninchens mit dem Enzym alkalische Phosphatase erhalten. Somit ist es möglich, das $^{125}$J-IgG unter Nutzung der entsprechenden Antikörperaktivitäten zur Reinigung und Isolierung des Enzyms alkalische Phosphatase einzusetzen. Hierzu wird durch n-Butanol-Extraktion vorgereinigte alkalische Phosphatase an die entsprechend Beispiel 6 mit $^{125}$J-IgG umgesetzte Sepharose gebunden und nachfolgend mit Triethylamin, pH 11,4, eluiert. Das so gereinigte Enzym hat eine spezifische Aktivität von 1000 IU/mg, wobei die Ausbeute ca 80 % beträgt. In der Polyacrylamid-Elektrophorese sind keine Begleitproteine nachweisbar.

Beispiel 10:

Humanes Immunglobulin G wird, wie in Beispiel 1 und 2 beschrieben, an Trisacryl® GF 2000 gebunden und zur Isolierung von Kaninchen anti-human-IgG-Antikörpern eingesetzt. Hierzu wird der mit dem IgG beladene Träger in eine Chromatographiesäule gefüllt, die mit einem spezifischen Antiserum gegen humanes IgG beladen wird.

Die Elution erfolgt zunächst mit PBS -Puffer und anschließend mit einer 3 m KSCN-Lösung. Nach sofortiger Dialyse der Antikörper-fraktionen wurde der Nachweis von spezifischen Antikörpern mit einem Enzymimmunoassay sowie der doppelten Immundiffusion (Ouchterlony-Technik) geführt. Die Prüfung auf unspezifische Bindungen erfolgt durch Auftragung eines entsprechenden Kaninchen-Normalserums sowie Testung aller antikörperhaltigen Fraktionen mit der Polyacrylamid-Disk-Elektrophorese.

Beispiel 11:

1 g Sepharose 4 B wird mit 5 % Mercaptopropyltrimethoxysilan (Serva, BRD ) in Ethanol/Wasser (1 : 1), 4 Stunden bei 60° C inkubiert und anschließend zweimal mit je 4 ml Ethanol/Wasser sowie fünfmal mit je 5 ml 0,1 m Phosphat-Puffer, pH 6,7 gewaschen. Anschließend wird das Gel mit 10 mg humanes IgG, das zuvor mit N-Succinimidyl 3-(2-pyridyldithio)propionat (SDPD, Pharmacia, Schweden) nach den Angaben des Herstellers aktiviert wurde, versetzt. Das bei der Reaktion freigesetzte Pyridin-2-thion kann photometrisch bei 343 nm verfolgt werden. Dieses so gebundene IgG kann analog Beispiel 10 zur Gewinnung und Isolierung von Kaninchen anti-human-IgG-Antikörpern eingesetzt werden. Nach Ablauf der Affinitätschromatographie kann das über Disulfidbrücken gebundene IgG durch Mercaptoethanol oder Dithiothreitol (50 mM) wieder abgespalten werden, so daß die aktivierte Matrix für eine erneute Bindung eines Liganden zur Verfügung steht.

Beispiel 12:

1 g makroporöses sphärisches Copolymer von 2-Hydroxyethylmethacrylat und Ethylendimethacrylat mit einer Partikelgröße von 15 -25 µm und einer inneren Oberfläche von ca. 70 $m^2/g$ Träger sowie einer Ausschlußgrenze von $2.10^6$ Dalton (im folgenden als Separon® Hema 1000 bezeichnet) wird mit 10 % Aminopropyltriethoxysilan (NB 1114 VEB Chemiewerk Nünchritz, DDR) in Ethanol/Wasser (1 : 1), pH 2,5; 6 Stunden bei 60° C und danach 6 Stunden bei 120° C inkubiert und anschließend zweimal mit je 5 ml Ethanol/Wasser sowie fünfmal mit je 5 ml 0,1 m Phosphat-Puffer, pH 6,8, gewaschen. Der Aktivierungsgrad wird nachfolgend durch Bestimmung der Aminogruppen am Träger ermittelt. Hierzu bietet sich die Methode

nach G. Antoni et al. (Anal. Biochem. 129 (1983)60) an. Der Aktivierungsgrad kann durch Wahl der Konzentration der Organosilane und/oder der Inkubationszeiten über einen weiten Bereich variiert werden. In der Regel erzielt man Aktivierungsgrade von 30 µmol/g Separon.

Beispiel 13:

5 g Separon Hema 1000 mit einer Partikelgröße von 15 -25 µm werden mit 10 % Aminopropyltriethoxysilan (NB 1114 VEB Chemiewerk Nünchritz, DDR) in Ethanol/Wasser (1 : 1), pH 2,5; 6 Stunden bei 60° C inkubiert und anschließend zweimal mit je 30 ml Ethanol/Wasser (1 : 1) bzw. fünfmal mit je 20 ml 0,1 m Phosphat-Puffer, pH 6,8 gewaschen. Das so aktivierte Gel wird nachfolgend mit 5 % igem Glutardialdehyd 2 Stunden bei 39° C in o,1 m Phosphat-Puffer, pH 6,8, umgesetzt und nachfolgend wiederum fünfmal mit je 20 ml Phosphat-Puffer gewaschen. Die Bindung des Proteins erfolgt durch einfach Zugabe der Proteinlösung zu dem auf diese Art und Weise aktivierten Gel. Hierzu wird das aktivierte Separon mit 10 ml humanes Immunglobulin in einer Konzentration von 18,6 mg/ml (0,1 m Phosphat-Puffer) versetzt und 2 Stunden bei 37° C oder über Nacht bei 4° C inkubiert. Nach Absaugen des nicht gebundenen Überstandes erfolgt die Bestimmung der gebundenen Proteinmenge am Träger durch Differenzmessung der Eiweißkonzentra tionen der eingesetzten Proteinlösung und des Überstandes. Auf diese Art und Weise konnten in jedem Experiment mindestens 95 % des angebotenen Proteins gebunden werden. In angeführtem Beispiel wurden von den angebotenen 186 mg IgG 183 mg Protein gebunden, so daß 36,7 mg IgG/g Separon gebunden sind.

Beispiel 14:

1 ml gequollenes Separon wird, wie in Beispiel 12 und 13 beschrieben, aktiviert und mit Glutaraldehyd in einer Konzentration von 3 % umgesetzt. Anschließend wird der so umgesetzte Träger mit 2 ml humanes Immunglobulin G in einer Konzentration von 35,5 mg/ml (= 71 mg) in einem 0,1 m Phosphat-Puffer, pH 6,8 2 Stunden bei Raumtemperatur versetzt. Nach Absaugen der überschüssigen Proteinmenge wird nachfolgend die gebundene Proteinmenge, wie in Beispiel 13 beschrieben, bestimmt. Auf diese Art und Weise konnten 23,6 mg IgG/ml Separon gebunden werden.

Beispiel 15:

1 ml gequollenes Separon wird mit einem Gemisch aus einem Aminopropyltriethoxysilan sowie einem Glycidoxypropyltriethoxysilan (Haftvermittler 6130, VEB Chemiewerk Nünchritz, DDR) in Kontakt gebracht und 2 Stunden bei Raumtemperatur geschüttelt. Nach Absaugen des überschüssigen Reagenz sowie Trocknen und nachfolgendem intensiven Waschen mit 0,1 m Phosphat-Puffer, pH 6,8, wird der Träger mit Glutaraldehyd umgesetzt und anschließend humanes igG-wie in Beispiel 14 beschrieben -gebunden. Es konnten 53,8 mg IgG/ml Separon gebunden werden.

Beispiel 16:

Humanes IgG wird, wie in Beispiel 12 -15 beschrieben, an Separon gebunden und zur Isolierung von Kaninchen anti-human-IgG-Antikörpern eingesetzt. Hierzu wird der mit dem IgG beladene Träger in eine Chromatographiesäule gefüllt, die mit einem spezifischen Antiserum gegen humanes IgG beladen wird. Die Elution erfolgt zunächst mit PBS-Puffer und anschließend mit einer 3 m KSCN-Lösung. Nach sofortiger Dialyse der Antikörperfraktionen wurde der Nachweis von spezifischen Antikörpern mit einem Enzymimmunoassay sowie der doppelten radialen Immundiffusion (Ouchterlony-Technik) geführt. Die Prüfung auf unspezifische Bindungen erfolgt durch Auftragung eines entsprechenden Kaninchen-Normalserums sowie Prüfung aller antikörperhaltigen Fraktionen mit der Polyacrylamid-Disk-Elektrophorese und Immunelektrophorese.

Beispiel 17:

1 g Perlzellulose mit einem Ausschlußvolumen von 5 000 000 D und einer Partikelgröße von 80 -200 µm sowie einem Trockensubstanzgehalt von 64 mg/ml wird mit 5% Aminopropyltriethoxysilan (NB 1114, VEB Chemiewerk Nünchritz, DDR) in Ethanol/Wasser (1 : 1), pH 2,5; 6 Stunden bei 60° C inkubiert und anschließend zweimal mit je 5 ml Ethanol/Wasser sowie fünfmal mit je 5 ml 0,1 m Phosphat-Puffer, pH 6,8 gewaschen. Nachfolgend wird der Aktivierungsgrad durch Bestimmung der Aminogruppen an der Festkörperoberfläche nach G.Antoni (Anal.Biochem. 129 (1983)60) bestimmt. Der Aktivierungsgrad kann durch Wahl der Konzentration der Organosilane und/oder der Inkubationszeiten iber einen weiten Bereich variiert werden. In der Regel erhält man charakteristische Aktivierungsgrade von 10 -15 µMol Aminogruppen je ml Perlzellulose.

Beispiel 18:

5 ml Perlzellulose entsprechend Beispiel 17 wird mit 10 % Aminopropyltriethoxysilan in Ethanol/Wasser ( 1 : 1), pH 2,5; 6 Stunden bei 60° C inkubiert und anschließend zweimal mit je 30 ml Ethanol/Wasser (1 : 1) bzw. fünfmal mit je 20 ml 0,1 Phosphat-Puffer, pH 6,8 gewaschen. Das so aktivierte Gel wird nachfolgend mit 5 %igem Glutaraldehyd 2 Stunden bei 37° C in o,1 m Phosphat-Puffer, pH 6,8, umgesetzt und nachfolgend wiederum fünfmal mit je 20 ml Phosphat-Puffer gewaschen. Anschließend erfolgt die Bindung des Proteins durch einfache Zugabe der Proteinlösung zu dem auf diese Art und Weise aktivierten Gel. Hierzu wird die Perlzellulose mit 10 ml humanem Immunglobulin G in einer Konzentration von 35,5 mg/ml in einem o,1 m Phosphat-Puffer, pH 6,8 bei 2 Stunden Raumtemperatur versetzt. Nach Absaugen des nicht gebundenen Überstandes erfolgt die Bestimmung der gebundenen Proteinmenge durch Differenzmessung der Eiweißkonzentration der eingesetzten Proteinlösung und des Überstandes. Auf diese Art und Weise konnten 12 mg IgG/ml Perlzellulose gebunden werden.

Beispiel 19:

1 ml Perlzellulose wird, wie in Beispiel 18 beschrieben, aktiviert und mit Glutaraldehyd in einer Konzentration von 3 % umgesetzt. Anschließend wird der so aktivierte Träger mit $^{125}$J-IgG (vom Kaninchen, gerichtet gegen das Enzym alkalische Phosphatase) in einer Konzentration von 41,3 mg/ml versetzt und 1 Stunde bei Raumtemperatur inkubiert. Nach Absaugen der überschüssigen Proteinmenge und nachfolgendem intensiven Waschen mit jeweils 5 ml Phosphat-Puffer wird die gebundene Proteinmenge durch Messung der Radioaktivität unter Mitführung der eingesetzten Proteinlösung als Standard bestimmt. In diesem Versuch konnten 11,4 mg $^{125}$J-IgG ml Perlzellulose gebunden werden.

Beispiel 20:

5 ml Perlzellulose entsprechend Beispiel 17 werden mit 5% Glycidoxypropyltriethoxysilan (NB 1115, VEB Chemiewerk Nünchritz, DDR) in Ethanol/Wasser (1 : 1), pH 2,5 sechs Stunden bei 60° C inkubiert und anschließend weitere sechs Stunden auf 120° C erhitzt. Nach intensivem Waschen mit je 20 ml Ethanol/Wasser bzw. 0,1 m Phosphat-Puffer analog Beispiel 18 wird die so aktivierte Perlzellulose mit 10 ml humanem Immunglobulin G in einer Konzentration von 35,5 mg/ml in einem 0,1 m Phosphat-Puffer, pH 6,8; zwei Stunden bei Raumtemperatur versetzt. Die gebundene Proteinmenge, die wie in Beispiel 18 beschrieben, bestimmt wurde, betrug 7,2 mg IgG/ml Perlzellulose.

Beispiel 21:

5 ml Perlzellulose werden entsprechend Beispiel 17 mit 5 % Mercaptopropyltrimethoxysilan (Serva, BRD) in Ethanol/Wasser (1 : 1), 4 Stunden bei 60° C inkubiert und anschließend mit je 5 ml Ethanol/Wasser sowie fünfmal mit je 5 ml 0,1 m Phosphat-Puffer, pH 6,8 gewaschen. Anschließend wird die Zellulose mit 10 mg humanem IgG, das zuvor mit N-Succinimidyl-3-(2-pyridyldithio)propionat (SDPD, Pharmacia, Schweden) nach den Angaben des Herstellers aktiviert wurde, versetzt. Das bei der Reaktion freigesetzte Pyridin-2-thion kann photometrisch bei 343 nm verfolgt werden. Dieses so gebundene IgG kann zur Gewinnung und

Isolierung von Kaninchen-anti-human-IgG-Antikörpern eingesetzt werden. Nach Ablauf der Affinitätschromatographie kann das über Disulfidbrücken gebundene IgG durch Mercaptoethanol oder Dithiothreitol (50 mM) wieder abgeschaltet werden, so daß die aktivierte Matrix für eine erneute Bindung eines Liganden zur Verfügung steht.

Beispiel 22:

5 ml Perlzellulose entsprechend Beispiel 17 werden mit einem Gemisch aus Aminopropyltriethoxysilan sowie Glycidoxypropyltriethoxysilan (Haftvermittler 6130, VEB Chemiewerk Nünchritz, DDR) in Kontakt gebracht und 2 Stunden bei Raumtemperatur geschüttelt. Nach Absaugen des überschüssigen Reagenz sowie Trocknen im Vakuum und nachfolgendem intensiven Waschen mit 0,1 m Phosphat-Puffer, pH 6,8 wird der Träger mit 3 % Glutaraldehyd umgesetzt und anschließend $^{125}$J-IgG, wie in Beispiel 19 beschrieben, gebunden. Es konnten 26,4 mg $^{125}$J-IgG/ml Perlzellulose gebunden werden.

Beispiel 23:

Die entsprechend den Beispielen 19 und 22 mit $^{125}$J-IgG umgesetzte Perlzellulose wird je dreimal mit 20 ml PBS-Puffer, pH 7,4,der Dioxan enthält, gewaschen und nachfolgend wird die gebundene Radioaktivität bestimmt. Die an der Perlzellulose verbliebenen Proteinmengen sind mit 11,4 mg/ml (Beispiel 19) und 26,6 mg/ml (Beispiel 22) unverändert.

Beispiel 24:

Das in den Beispielen 19, 22 und 23 eingesetzte Immunglobulin G wurde durch Immunisierung eines Kaninchens mit dem Enzym alkalische Phosphatase erhalten. Somit ist es möglich, das $^{125}$J-IgG unter Nutzung der entsprechenden Antikörperaktivitäten zur Reinigung und Isolierung des Enzyms alkalische Phosphatase einzusetzen. Hierzu wird durch n-Butanol-Extraktion vorgereinigte alkalische Phosphatase an die entsprechend Beispiel 19 mit $^{125}$J-IgG umgesetzte Perlzellulose gebunden und nachfolgend mit Triethylamin, pH 11,4, eluiert. Das so gereinigte Enzym hat eine spezifische Aktivität von 1 000 IU/mg, wobei die Ausbeute ca. 80 % beträgt, in der Polyacrylamidelektrophorese sind keine Begleitproteine nachweisbar.

Beispiel 25:

Humanes Immunglobulin G wird, wie in Beispiel 18 beschrieben, an Perlzellulose gebunden und zur Isolierung von Kaninchen-anti-human-IgG-Antikörpern eingesetzt. Hierzu wird der mit dem IgG beladene Träger in eine Chromatographiesäule gefüllt, die mit einem spezifischen Antiserum gegen humanes IgG beladen wird. Die Elution erfolgt zunächst mit PBS-Puffer und anschließend mit einer 3 m KSCN-Lösung. Nach sofortiger Dialyse der Antikörperfraktionen wurde der Nachweis von spezifischen Antikörpern mit einem Enzymimmunoassay sowie der doppelten radialen Immundiffusion (Ouchterlony-Technik) geführt. Die Prüfung auf unspezifische Bindungen erfolgt durch Auftragung eines entsprechenden Kaninchen-Normalserums sowie Testung aller antikörperhaltigen Fraktionen mit der Polyacrylamid-Disk-Elektrophorese.

Beispiel 26:

Flächenförmige Zelluloseträger (Whatman-Papier 560 und FN 4-Papier) werden mit 3 % Aminopropyltriethoxysilan oder Glycidoxypropylsilan, wie in Beispiel 19 und 20 beschrieben, aktiviert und anschließend mit $^{131}$J-Human-serumalbumin ($^{131}$ J-HSA) in o,1 m Phosphat-Puffer, pH 6,8, in den Konzentrationen 10 µg/ml und 100 µg/ml versetzt. Nach intensivem Waschen wurde die gebundene Proteinmenge durch Messung der Radioaktivität unter Mitführung eines Standards bestimmt. Die hierbei erhaltenen Ergebnisse sind in Tab.1 zusammengefaßt

Tabelle 1

| | gebundenes $^{131}$ J-HSA | | | |
|---|---|---|---|---|
| | Aminopropyltriethoxysilan | | Glycidoxypropyltriethoxysilan | |
| eingesetzte Proteinkonz. | 10 µg/ml | 100 µg/ml | 10 µg/ml | 100 µg/ml |
| FN 4 | 192 ng/cm² | 1036 ng/cm² | 75 ng/cm² | 343 ng/cm² |
| Whatman 580 | 61 ng/cm² | 486 ng/cm² | 15 ng/cm² | 76 ng/cm² |

Beispiel 27:

Die in Beispiel 26 mit $^{131}$ J-HSA beladenen Papierträger werden im folgenden im Enzymimmunoassay eingesetzt. Hierzu werden definierte Flächen dieser Träger nach vorherigem Blocken eventuell noch freier reaktiver Gruppen mit Äthanolamin mit einem Kaninchen-anti-human-Albumin-Antiserum in geeigneter Verdünnung versetzt und bei Raumtemperatur über Nacht inkubiert. Nach dreimaligem Waschen zur Entfernung der nicht gebundenen Antikörper werden die Papierträger mit einem Ziege-anti-Kaninchen-IgG-Konjugat (Enzym: alkalische Phosphatase) 4 Stunden bei 37 ° C inkubiert und erneut dreimal gewaschen. Die Enzymaktivität wird durch Hydrolyse von 4-Nitrophenylphosphat in 0,5 ml Diethanolamin-Puffer, pH 9,8 und photometrische Messung des Enzymproduktes bei 405 nm bestimmt.

Beispiel 28:

Flächenförmige Zelluloseträger (Whatman 560, Filtrak 1389, Filtrak 390, FN 3) werden, wie in Beispiel 22 beschrieben, mit einem Gemisch aus Aminopropyltriethoxysilan und Glycidoxypropyltriethoxysilan aktiviert und anschließend mit $^{125}$J-IgG (gerichtet gegen das Enzym alkalische Phosphatase) in zwei Konzentrationen in 0,1 m Phosphat-Puffer, pH 6,8, inkubiert. Nach dreimaligem Waschen mit einem PBS-Puffer wird die gebundene Proteinmenge durch Messung der Radioaktivität bestimmt. Danach werden die flächenförmigen Zelluloseträger erneut zehnfach mit PBS-Puffer gewaschen und die gebundene Proteinmenge wiederum durch Messung der Radioaktivität unter Mitführen eines Standards bestimmt. Schließlich werden die Zelluloseträger über einen Zeitraum von vier Wochen mehrfach mit HCl-Glycin-Puffer, pH 2,2, 3m KSCN, Carbonat-Bicarbonat-Puffer, pH 10, und PBS-Puffer, 20 % Dioxan enthaltend, gewaschen und erneut zur Bestimmung der gebundenen Proteinmenge eingesetzt.

Die Ergebnisse sind in Tab.2 dargestellt:

Tabelle 2

| | gebundenes $^{125}$J-IgG | | | |
|---|---|---|---|---|
| | Whatman 560 | Filtrak 1389 | Filtrak 380 | FN 3 |
| gebundene Proteinmenge nach dreimaligem Waschen: | | | | |
| 10 µg/ml | 2092 ng/cm² | 2143 ng/cm² | 2015 ng/cm² | 2372 ng/cm² |
| 50 µg/ml | 5536 ng/cm² | 6122 ng/cm² | 5867 ng/cm² | 5867 ng/cm² |
| gebundene Proteinmenge nach weiteren 10 Waschvorgängen: | | | | |
| 10 µg/ml | 1888 ng/cm² | 1939 ng/cm² | 1796 ng/cm² | 2092 ng/cm² |
| 50 µg/ml | 5000 ng/cm² | 5612 ng/cm² | 5281 ng/cm² | 5459 ng/cm² |
| gebundene Proteinmenge nach vier Wochen: | | | | |
| 10 µg/ml | 1913 ng/cm² | 1913 ng/cm² | 1786 ng/cm² | 1964 ng/cm² |
| 50 µg/ml | 5102 ng/cm² | 5612 ng/cm² | 5306 ng/cm² | 5281 ng/cm² |

Beispiel 29:

Ein Flächenträger mit papierähnlicher Matrix gemäß der Erfindung wird mit human Immunglobulin G, welches mit $^{125}$J radioaktiv markiert ist, in verschiedenen Konzentrationen und Puffersystemen versetzt und 16 Stunden bei 37° C inkubiert. Nach dreimaligem Waschen mit PBS-Puffern, pH 7,4, der 0,025 % Tween 20 enthält, werden die eventuell noch überschüssigen reaktiven Gruppen mit 1 m Äthanolaminlösung blockiert. Nach erneutem Waschen mit obigen PBS-Puffern wird die gebundene Proteinmenge durch Messung der Radioaktivität ermittelt. Zur Ermittlung möglicher Desorptionen werden die Proben anschließend erneut siebenmal mit PBS-Puffer gewaschen. Die so beladenen Flächenträger werden danach zur Ermittlung der Immunreaktivität mit einem Konjugat bestehend aus einem Kaninchen-anti-human IgG und dem Enzym alkalische Phosphatase versetzt und iber Nacht bei Raumtemperatur inkubiert. Nach erneutem Waschen wird die Enzymaktivität der alkalischen Phosphatase durch Hydrolyse von 4-Nitrophenylphosphat in 0,5 ml Diethanolaminpuffer pH 9,8 und photometrische Messung des Enzymproduktes bei 405 nm nach Stoppen mit 1 n NaOH oder 1 n EDTA bestimmt. Nach Ausführung des Enzymimmunoassay werden die Flächenträger zweimal mit je 0,5 ml PBS-Puffer gewaschen und nachfolgend in Mehrfachbestimmungen mit einer der folgenden Dissoziationsreagenzien für 1 h bei 4° C versetzt.

1) PBS-Puffer, 2 m an NaCl, 5 % Dioxan enthaltend, 2) 1 m KSCN, 3) 3 m KSCN, 4) 4,5 m $MgCl_2$, 5) 1 m NaJ, 6) HCl-Glyzin-Puffer pH 2,8.

Durch alle diese Agenzien werden die gebundenen Antigen-Antikörper-Komplexe gespalten, so daß die mit humanem IgG beladenen Flächenträger erneut im Enzymimmunoassay eingesetzt werden können.

Beispiel 30:

Flächenförmige erfindungsgemäße Formkörper (F = 56 $mm^2$) werden, wie in Beispiel 22 beschrieben, aktiviert und mit humanem Faktor VIII beladen. Diese Träger werden in Polystyrol-Mikrotitrations-Platten oder Polystyrol-Röhrchen überführt und im folgenden der Enzymimmunoassay zur Bestimmung von F VIII-Antigen (W. Schlößler, M. Stepanauskas, Chr. Dittrich, H. Heine; Acta biol.med.germ.41 (1982) 263; W. Schlößler, M.Stepanauskas, Chr. Dittrich: Acta biol. med,germ 41 (1982) 695) in der beschriebenen Art und Weise durchgeführt. Hierzu werden die Formkörper mit einem Inkubationsgemisch, bestehend aus dem zu bestimmenden Plasma und einem im Überschuß vorhandenen Kaninchen-anti-human-Faktor VIII-Antikörper, versetzt und 6 Stunden bei 37° C inkubiert. Nach erneutem Waschen mit PBS-Puffer, pH 7,4, der 0,05 % Tween 20 enthält, werden 200 µl eines Konjugates, bestehend aus einem Hammel-anti-Kaninchen-IgG und dem Enzym alkalische Phosphatase, zugegeben und nach mehrstündiger Inkubation und nachfolgendem Auswaschen die Enzymaktivität, wie in Beispiel 29 beschrieben, bestimmt. Die Abspaltung der gebundenen Antikörper erfolgt mit einem der in Beispiel 29 angeführten Dissoziationsreagenzien, so daß die mit Faktor VIII beladenen Träger erneut im Immunoassay eingesetzt werden können.

Beispiel 31:

Ein erfindungsgemäßer Flächenträger mit papierähnlicher Matrix wird, wie in Beispiel 30 beschrieben, mit humanem Faktor VIII beladen. Dieser Träger dient im folgenden als Modell für screening-Untersuchungen auf (monoklonale) Antikörper. Nach Ausführung der Waschvorgänge, die sich bei diesem Flächenträger besonders einfach gestalten, werden die zu untersuchenden und zu bestimmenden Substanzen mit einem geeigneten Auftragsstempel punktförmig aufgetragen. In unserem Fall dienten hierzu geeignete Verdünnungen eines Kaninchen-anti-human-Faktor VIII-Antiserums bzw. eines Kaninchenserums als Negativkontrolle als Antikörperquelle. Nach einer Inkubation von vier bis sechs Stunden bei 37° C wird der Flächenträger erneut gewaschen und mit einem Schaf-anti-Kaninchen-IgG, gekoppelt mit dem Enzym Peroxydase, vier Stunden bei 37° C oder über Nacht bei Raumtemperatur inkubiert, erneut mehrmals gewaschen und die Enzymaktivität mit einem geeigneten Nachweissystem, so zB. 4 mM O-Phenylendiamin und 1,5 mM $H_2O_2$ durch visuelle Auswertung der entstehenden Färbung bestimmt. Eine positive Färbung weist eindeutig auf einen Antikörper hin.

Beispiel 32:

$^{32}$P-markierte Phosphoproteine, die in Membranvesikeln aus Herzmuskel enthalten sind, werden in dem phosphat-gepufferten Natriumdodecylsulfat-Polyacrylamid-Gelelektrophoresesystem nach Weber et al. (K.Weber, J. Pringle, M. Osborn, Meth. Enzymol. 26, (1972) 3) aufgetrennt. Sofort nach Beendigung der Elektrophorese werden die aufgetrennten Proteine auf das erfindungsgemäß aktivierte Papier in einem Triethanolamin und Buttersäure enthaltenden Transfersystem nach Kyhse-Andersen (J.Biochem, Biophys. Meth. 10 (1984) 203) dem Immunoblotting unterworfen. Nach dem Blotting wird in phosphatgepufferter physiologischer Kochsalzlösung, die 0,1 % Gelatine und 0,05 % Tween enthielt, gebadet, anschließend mit einem ersten Kaninchen-Antiserum inkubiert, dem eine Inkubation mit einem Anti-Kaninchen-Immunoglobulin-Meerrettichperoxidase-Konjugat und schließlich die Indikatorreaktion folgt. An Stelle des Peroxidase-Konjugates kann mit gleichem Erfolg ein alkalische-Phosphatase-Konjugat verwendet werden.

Beispiel 33:

Die Proteine intrazellulärer Membranen verschiedener Muskelzelltypen werden in einem Dodecylsulfat-Polyacrylamid-Gelelektrophorese-System nach Laemmli (U.K. Laemmli, Nature 227, (1970) 680) aufgetrennt. Nach der Elektrophorese wird das Gel für 20 bis 60 Min. in einer Pufferlösung, bestehend aus 25 mM Triethanolamin-Hydrochlorid, pH 8,4, 0,1 % Natriumdodecylsulfat, gebadet. Daran schließt sich unmittelbar das Immunoblotting, wie in Beispiel 32 beschrieben, an.

## Ansprüche

1. Verfahren zur Aktivierung von Hydroxyl-Gruppen enthaltenden natürlichen und synthetischen, polymeren Festkörperoberflächen, gekennzeichnet dadurch , daß die Hydroxyl-Gruppen mit einem Organosilan der Formel

$(XR'_{n'})_n$ Si $R_{4-n}$ (I)

umgesetzt werden, wo X eine Amino-, Carbonyl-,Carboxy-, Epoxy-, Isocyano-, Diazo-, Isothiocyano-, Nitroso-, Sulfhydryl-oder Halocarbonyl-Gruppe und R′ eine Alkyl-, Alkylphenyl-oder Phenyl-Gruppe und R eine Alkoxy-, Phenoxy-oder Halogen-Gruppe sind, wobei n′ den Wert zwischen 0 -20 und n den Wert zwischen 1-3 besitzen, oder daß die Umsetzung mit mindestens zwei Organosilanen der Formel I im Gemisch oder nacheinander in flüssiger oder gasförmiger Phase erfolgt und daß anschließend gegebenenfalls eine weitere Behandlung mit homo-oder heterobifunktionellen Reagenzien durchgeführt wird.

2. Verfahren nach Anspruch 1, gekennzeichnet dadurch, daß die Umsetzung der Hydroxyl-Gruppen mit Verbindungen der Formel I in organischen Lösungsmitteln, Lösungsmittelgemischen, wässrigen Lösungen oder Gemischen aus Wasser und organischen Lösungsmitteln erfolgt.

3. Verfahren nach Anspruch 1, gekennzeichnet dadurch, daß die Umsetzung der Hydroxyl-Gruppen in der Gasphase mit einem Aerosol oder bei einem Druck unterhalb des Atmosphärendrucks erfolgt.

4. Verfahren nach den Ansprüchen 1 bis 3, gekennzeichnet dadurch, daß die Polymere als Formkörper vorliegen.

5. Verfahren nach Anspruch 4, gekennzeichnet dadurch, daß die Formkörper eine sphärische, faserige oder eckige Form besitzen.

6. Verfahren nach Anspruch 4, gekennzeichnet dadurch, daß die Formkörper kugelförmig sind.

7. Verfahren nach einem der Ansprüche 4 bis 6, gekennzeichnet dadurch, daß die Formkörper durch Packung, Verweben oder durch Bindemittel zu Kolonnenpackungen oder flächenförmigen Materialien zusammengestellt sind.

8. Verfahren nach Anspruch 4, gekennzeichnet dadurch, daß die Formkörper als dünne Schichten vorliegen, die als Filme oder Lacke auf anderen Trägern befindlich sind.

9. Aktivierte Polymerfestkörper, bestehend aus organischen, OH-gruppenhaltigen Makromolekülen - (hergestellt mit dem Verfahren nach einem der Ansprüche 1 bis 8), gekennzeichnet dadurch, daß die Oberfläche der Polymerfestkörper Gruppierungen der allgemeinen Formel

$(\text{-O-})_{4-n}$ Si $(R'_{n'}X)_n$ IV

wobei R′ eine Alkyl-, Alkylphenyl-oder Phenyl-Gruppe, und X eine Amino-, Carbonyl-, Carboxy-, Epoxy-, Isocyano-, Diazo-, Isothiocyano-, Nitroso-, Sulfhydryl-oder Halocarbonyl-Gruppe ist und n′ den Wert von 0 - 20 und n 1 -3 besitzen, und OH-Gruppen aufweisen, die zur Gruppierung IV im Verhältnis von 1 : 9 bis 9 : 1 stehen.

10. Aktivierte Polymerfestkörper nach Anspruch 9, gekennzeichnet dadurch, daß die Polymerfestkörper eine sphärische, faserige oder eckige Form besitzen.

11. Aktivierte Polymerfestkörper nach Anspruch 9 oder 10, gekennzeichnet dadurch, daß die Polymerfestkörper im gebundenen und gepackten Zustand eine vorwiegend zweidimensionale Ausdehnung aufweisen.

12. Aktivierte Polymerfestkörper nach Anspruch 11, gekennzeichnet dadurch, daß die gebundenen Polymerfestkörper Vliese, Papiere, Folien, Filme oder Lacke sind.